# EUROPEAN PATENT APPLICATION

(11) **EP 3 939 837 A1**
(43) Date of publication of application: **19.01.2022**
(21) Application number: 20808637.1
(22) Date of filing: 27.04.2020
(51) Int. Cl.: B60R 11/04, B60R 16/02, G06F 3/01

(54) **CONTROL SYSTEM AND PRESENTATION SYSTEM**

(30) Priority: 17.05.2019 JP 2019093981; 03.04.2020 JP 2020067514
(71) Applicant: Kabushiki Kaisha Tokai Rika Denki Seisakusho, Aichi 480-0195 (JP)
(72) Inventor: MITSUI, Yuji, Niwa-gun, Aichi 480-0195 (JP); SASAKI, Shinobu, Niwa-gun, Aichi 480-0195 (JP); IWATA, Kenji, Niwa-gun, Aichi 480-0195 (JP); OHNISHI, Takeshi, Niwa-gun, Aichi 480-0195 (JP); TAKAGI, Yuka, Niwa-gun, Aichi 480-0195 (JP); HIROSE, Fumiaki, Niwa-gun, Aichi 480-0195 (JP); TAKAI, Toshihito, Niwa-gun, Aichi 480-0195 (JP); ARAYA, Takao, Niwa-gun, Aichi 480-0195 (JP); NOMURA, Keiji, Niwa-gun, Aichi 480-0195 (JP); NAKANE, Keita, Niwa-gun, Aichi 480-0195 (JP); SENGOKU, Takakazu, Niwa-gun, Aichi 480-0195 (JP); IMAI, Tomomi, Niwa-gun, Aichi 480-0195 (JP)
(74) Representative: TBK
(86) International application number: PCT/JP2020/017884
(87) International publication number: WO 2020/235306

(57) **Abstract**

Information presentation that enables a subject to recognize information more easily is performed. A control system includes a control unit that controls presentation of information in a presentation unit that presents the information, in which the control unit controls a mode of the presentation in the presentation unit according to the information to be reported to a subject.

## Description

### Technical Field

The present invention relates to a control system and a presentation system.

### Background Art

In recent years, devices for notifying a subject who operates a moving object (for example, an automobile or the like) of information such as the occurrence of an abnormality through tactile presentation have become widespread. For example, the following Patent Literature 1 discloses a driving support device that causes a subject to pay attention to a traveling environment on the basis of a notification using vibration.

### Citation List

### Patent Literature

Patent Literature 1: JP 2015-001776A

### Summary of Invention

### Technical Problem

However, information to be reported to a subject is not necessarily uniform. Therefore, when tactile presentation is performed in the same manner for all information, inconvenience that a subject is confused or information is misrecognized may occur.

Therefore, the present invention has been made in light of the above problem, and an object of the present invention is to provide a control system and a presentation system that are novel and improved, capable of performing presentation that enables a subject to recognize information more easily.

### Solution to Problem

According to an aspect of the present invention, there is provided a control system comprising a control unit that controls presentation of information in a presentation unit that presents the information, wherein the control unit controls a mode of the presentation in the presentation unit according to the information to be reported to a subject.

The information may include first information and second information, and the control unit may control presentation of the information in the presentation unit such that the first information and the second information are respectively presented in a first presentation mode and a second presentation mode different from the first presentation mode.

The presentation unit may have a plurality of different types of presentation elements, and wherein the control unit may realize the first presentation mode and the second presentation mode with different presentation elements.

The control unit may realize the first presentation mode and the second presentation mode with presentation elements of the same type.

The first information and the second information may be respectively acquired from different information sources.

The first information and the second information may be acquired from the same information source.

The first information and the second information may be the same type of information.

The first information and the second information may be different types of information.

According to another aspect of the present invention, there is provided a presentation system comprising a presentation unit that presents information, wherein the presentation unit performs presentation of the information in different modes according to the information to be reported to a subject on the basis of an input control signal.

### Advantageous Effects of Invention

According to the above present invention, it is possible to perform presentation that enables a subject to recognize information more easily.

### Brief Description of Drawings

[FIG. 1] FIG. 1 is an explanatory diagram illustrating a configuration of a control system 10 according to an embodiment of the present invention.
[FIG. 2] FIG. 2 is a flowchart illustrating an example of the control system 10 according to the embodiment of the present invention.
[FIG. 3] FIG. 3 is a flowchart illustrating an operation according to an application example.

### Description of Embodiments

Hereinafter, embodiments of the present invention will be described in detail with reference to the accompanying drawings. In the specification and the drawings, constituents having substantially the same functional configuration are given the same reference numerals, and thus repeated description will be omitted.

### <1. Outline>

First, an outline of an embodiment of the present invention will be described. In recent years, techniques for causing a subject to recognize information through tactile presentation have been proposed. Such techniques may be applied to, for example, information (such as alerting and warning) regarding a traveling environment of a driver who is driving any of various moving objects such as a vehicle.

Such information of which it is desirable to notify a driver through tactile presentation is not limited to the information regarding a traveling environment. For example, in recent years, with technological progress, automation of a driving system has progressed. Here, a driver of a moving object of which driving is automated (semi-automated) tends not to be engaged in the driving task. It is known that the degree of alertness of a driver who is no engaged in a driving task is reduced. However, in a situation in which automated driving and manual driving are mixed, as in level 3 defined by the SAE International, a driver is required to maintain a certain degree of alertness in order to reliably respond to a take over request (TOR) from a system.

Therefore, in a case where the degree of alertness of a driver is reduced, it is desirable to notify the driver of the reduction of the degree of alertness and to perform tactile presentation for stimulating alertness in the driver.

However, if tactile presentation is performed in the same manner for information of different types and purposes, such as information regarding a traveling environment and information regarding an alertness state, inconvenience that a driver is confused or information is misrecognized may occur.

The present inventor has created an embodiment of the present invention with the above circumstances as one point of view. A control system according to the embodiment of the present invention includes a control unit that controls presentation of information in a presentation unit that presents the information, and the control unit controls a presentation mode for presenting the information in the presentation unit according to the information to be presented to a subject.

In the present specification, a driver who mainly drives a moving object will be described as the above subject. However, the subject is not limited to a driver, and may be another person who wants to remain alert (for example, a student taking a class, a participant in a conference, a worker at work, and a player of a game). The subject is not limited to a human, and may be an animal such as a dog, a horse, or a monkey.

In the present specification, a real vehicle will be mainly described as the above moving object. However, a concept of the moving object is not limited to a real vehicle. That is, the concept of the moving object includes real or virtual vehicles, aircrafts, or ships. More specifically, examples of the moving object include real vehicles (for example, an automobile, a bus, a bike, a locomotive, or a train), aircraft (for example, an airplane, a helicopter, a glider, or an airship), and ships (for example, a passenger ship, a cargo ship, or a submarine). The moving object may include a virtual vehicle, aircraft, or ship, for example, a simulator or a game machine related to a vehicle, an aircraft, or a ship. Examples of the moving object may include not only manned objects but also unmanned objects. For example, the moving object may be an unmanned transport vehicle, an unmanned aircraft, an unmanned ship, or a radio-controlled object that is operated remotely by a subj ect.

The above-described tactile presentation is presentation of a stimulus that acts on the sense of touch of a subject. For example, tactile presentation may include tactile presentation using a vibration stimulus, tactile presentation using an electrical stimulus, tactile presentation using a temperature change, tactile presentation related to a force sensation (for example, presentation of a sensation of being pushed by an object, presentation of a sensation of coming into contact with an object, or presentation of tightening) or tactile presentation related to a skin sensation (for example, presentation of a rough sensation or presentation of a slippery sensation).

Hereinafter, a configuration and an operation of a control system according to an embodiment of the present invention will be sequentially described in detail.

### <2. Configuration of control system>

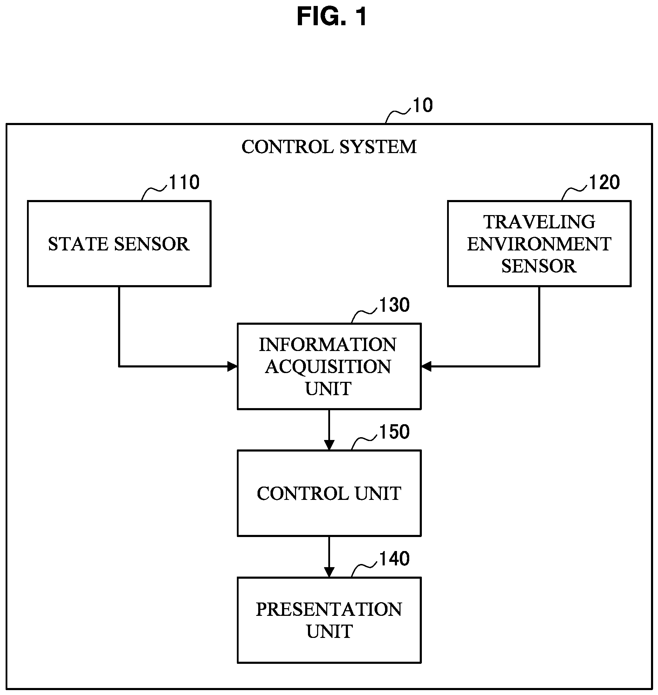

FIG. 1 is an explanatory diagram illustrating a configuration of a control system 10 according to an embodiment of the present invention. As illustrated in FIG. 1, the control system 10 according to the embodiment of the present invention includes a state sensor 110, a traveling environment sensor 120, an information acquisition unit 130, a presentation unit 140, and a control unit 150.

### (State sensor 110)

The state sensor 110 is a sensor that continuously detects various states of a driver. The state sensor 110 may be a camera that images the driver's face, an optical sensor, a temperature sensor, or a pulse sensor that detects the heartbeat or a skin temperature of the driver, or may be a combination thereof.

### (Traveling environment sensor 120)

The traveling environment sensor 120 is a sensor that continuously detects a traveling environment such as the current position of a vehicle driven by a driver and a distance to another vehicle. The traveling environment sensor 120 may be a position estimation device such as a Global Positioning System (GPS) sensor that estimates the current position of the vehicle, or a distance measurement device that measures the distance to another vehicle, or may be a combination thereof.

### (Information acquisition unit 130)

The information acquisition unit 130 acquires information to be presented to a subject. For example, as information to be reported to a driver, the information acquisition unit 130 acquires information regarding the driver state, information regarding a traveling environment, useful information that is useful for a user, information regarding a surrounding environment related to a surrounding status, and the like.

The information acquisition unit 130 estimates a driver state on the basis of a result of detection by the state sensor 110, and acquires information regarding the driver state. For example, the information acquisition unit 130 estimates the degree of alertness of the driver on the basis of a result of detection by the state sensor 110, and acquires, as information to be reported, information indicating a decrease in the degree of alertness in a case where the degree of alertness of the driver is less than a threshold value. Specifically, the information acquisition unit 130 may detect an open/closed state of the driver's eyelids on the basis of an image of the driver acquired by the state sensor 110 through imaging, and estimate the degree of alertness on the basis of the open/closed state. More specifically, the information acquisition unit 130 may calculate an eye-closing ratio, an eye-opening ratio, or the like on the basis of the image of the driver, and use the eye-closing ratio, the eye-opening ratio, or the like as the degree of alertness. The information acquisition unit 130 may estimate the degree of alertness of the driver from the driver's heartbeat or skin temperature detected by the state sensor 110.

In the present specification, an example in which the information acquisition unit 130 estimates the degree of alertness of a driver on the basis of a result of detection by the state sensor 110 will be mainly described, but information regarding the driver state acquired by the information acquisition unit 130 is not limited to information indicating the degree of alertness. For example, the information acquisition unit 130 may acquire information regarding the driver state such as a driver's inattentiveness, a driver's distractedness, a driver's unconsciousness, or a driver's drinking condition on the basis of the result of detection by the state sensor 110.

The information acquisition unit 130 acquires information regarding a traveling environment on the basis of a result of detection by the traveling environment sensor 120. For example, the information acquisition unit 130 estimates the presence or absence of a specific event on the basis of the result of detection by the traveling environment sensor 120, and, when a specific event occurs, acquires information indicating the specific event (caution or warning) as information to be reported. Here, the information acquisition unit 130 may estimate the presence or absence of a specific event in which a traveling route of a vehicle is likely to deviate from a predetermined traveling route on the basis of the current position of the vehicle detected by the traveling environment sensor 120. The information acquisition unit 130 may estimate the presence or absence of a specific event in which a distance to another vehicle is equal to or less than a threshold value on the basis of a distance to the other vehicle detected by the traveling environment sensor 120.

The functions of the information acquisition unit 130 may be realized in cooperation with, for example, a processor such as a central processing unit (CPU) or a micro controller unit (MCU), software, and a storage medium such as a read only memory (ROM) or a random access memory (RAM). Similarly, functions of the control unit 150 that will be described later may be realized in cooperation with a processor, software, and a storage medium.

A method of the information acquisition unit 130 acquiring information is not limited to the above-described example. For example, the information acquisition unit 130 may acquire information from a car navigation system, a roadside machine, another vehicle, a server, or the like. Information may be acquired from the roadside machine by using well-known road-to-vehicle communication, standard road-to-vehicle communication, or the like. Information may be acquired from another vehicle by using well-known vehicle-to-vehicle communication, standard road-to-vehicle communication, or the like. Information may be acquired from the server by using well-known wireless communication, standard wireless communication, or the like.

The information acquired by the information acquisition unit 130 is not limited to the above-described example. For example, the information acquisition unit 130 may acquire useful information, information regarding a surrounding environment, and the like. The useful information may be information desired by a subject and designated by the subject, or information not designated by the subject but useful for the subject. The information regarding the surrounding environment may be a relative change in physical quantity, the degree of vector divergence, or information according to a level of attention.

### (Presentation unit 140)

The presentation unit 140 presents information to a subject under the control of the control unit 150. In an embodiment of the present invention, the presentation unit may have presentation elements for any of tactile presentation, visual presentation, olfactory presentation, gustatory presentation, or auditory presentation, or a plurality of presentation elements of different types. Examples of a tactile presentation element for tactile presentation include a configuration in which tactile presentation is performed by using a vibration stimulus, a configuration in which tactile presentation is performed by using an electrical stimulus, a configuration in which tactile presentation is performed by using a temperature change, tactile presentation related to a force sensation (for example, presentation of a sensation of being pushed by an object, presentation of a sensation of coming into contact with an object, or presentation of tightening), and a configuration in which tactile presentation related to a skin sensation (for example, presentation of a rough sensation or presentation of a slippery sensation) is performed.

Examples of the visual presentation element for visual presentation include a configuration having a lighting function and a configuration capable of changing the light transmittance such as dimming glass. Examples of the olfactory presentation element for olfactory presentation includes a configuration in which a scent is diffused in the air. Examples of the gustatory presentation element for gustatory presentation include a configuration for adjusting a taste of a substance to be placed in the oral cavity, and a configuration for adjusting a taste of a substance already placed in the oral cavity. Examples of the auditory presentation element for auditory presentation include a configuration for generating air vibration, such as a speaker or an earphone, and a configuration having a bone conduction function.

As a more specific example of the tactile presentation element, the tactile presentation element may include a plurality of tactile presentation elements at different installation locations. For example, the plurality of tactile presentation elements may include two or more tactile presentation elements among a vibration device worn on the driver's hand, a vibration device in close contact with the driver's waist, a seatbelt winding motor, a vibrator installed on the vehicle floor, a brake, a vibrator installed in a seat, and a vibrator installed in a steering wheel. Operation parameters of each presentation element may be adjustable. For example, operation parameters such as a strength, a waveform, a length of time, and an interval of vibration generated by the tactile presentation element may be adjustable.

The plurality of tactile presentation elements may include tactile presentation elements having different tactile presentation methods. For example, the plurality of tactile presentation elements may include a non-contact tactile presentation element that performs tactile presentation to the driver in a non-contact state, and a contact type tactile presentation element that performs tactile presentation to the driver in a contact state. Non-contact tactile presentation elements include an ultrasonic vibration generation device that is installed on a center console or a door and emits ultrasonic vibration toward the thighs of the driver's feet, an air extrusion device that pushes air toward the driver's face, and a far-infrared emitting device that radiates far-infrared rays to the driver.

### (Control unit 150)

The control unit 150 controls a mode of presentation performed by the presentation unit 140 in order to present information according to the information acquired by the information acquisition unit 130. Specifically, the control unit 150 controls the presentation unit 140 such that presentation of first information is executed in a first presentation mode, and presentation of second information is executed in a second presentation mode different from the first presentation mode.

### - First presentation mode and second presentation mode

The first presentation mode and the second presentation mode described above are not particularly limited. For example, the first presentation mode and the second presentation mode may be presentation modes realized by different presentation elements, or may be presentation modes realized by the same type of presentation elements.

A combination of different presentation elements may be a combination of two of tactile presentation, visual presentation, olfactory presentation, gustatory presentation, and auditory presentation. In a case of tactile presentation using the same the type of presentation elements, a combination of the same type of presentation elements may be a combination of two of a configuration in which tactile presentation is performed by using a vibration stimulus, a configuration in which tactile presentation is performed by using electrical stimulus, a configuration in which tactile presentation is performed by using a temperature change, a configuration in which tactile presentation related to a force sensation is performed, and a configuration in which tactile presentation related to a skin sensation is performed.

Such a combination of the first presentation mode and the second presentation mode may be a combination in which either the first presentation mode or the second presentation mode includes tactile (vibration) presentation. For example, the first presentation mode may be realized by a tactile presentation element, and the second presentation mode may be realized by a tactile presentation element, an auditory presentation element, a gustatory presentation element, an olfactory presentation element, or a visual presentation element. The first presentation mode may be realized by a combination of a tactile presentation mode and another type of presentation element (for example, an auditory presentation element or a visual presentation element), and the second presentation mode may be realized by a tactile presentation element.

The first presentation mode may be realized by a plurality of different types of tactile presentation elements, and the second presentation mode may also be realized by the tactile presentation elements. The plurality of different types of tactile presentation elements may be, for example, a combination of a configuration in which tactile presentation is performed by using a vibration stimulus and a configuration in which tactile presentation is performed by using an electrical stimulus, or a combination of a configuration in which tactile presentation is performed by using a contact type vibration stimulus and a configuration in which tactile presentation is performed by using a non-contact type vibration stimulus.

Each of the first presentation mode and the second presentation mode may be a combination of presentations performed by a plurality of presentation elements. Both of the first presentation mode and the second presentation mode may be realized by tactile presentation elements and other presentation elements of the same type (such as auditory presentation elements or visual presentation elements). Specifically, the first presentation mode may be presentation performed by a combination of a tactile presentation element and an auditory presentation element, and the second presentation mode may also be presentation performed by a combination of a tactile presentation element and an auditory presentation element. In this case, a volume of sound presented by the auditory presentation element, a waveform of the sound, and the like may be different between the first presentation mode and the second presentation mode. Alternatively, in the first presentation mode and the second presentation mode, the operation parameters such as the strength, the waveform, the length of time, and the interval of vibration described above in tactile presentation elements may be different. Alternatively, in the first presentation mode and the second presentation mode, the operation parameters such as the strength, the waveform, the length of time, and the interval of vibration described above in the tactile presentation elements are different, and in the first presentation mode and the second presentation mode, the volume of the sound presented by auditory presentation elements, the waveform of the sound, and the like may be different.

In a case where tactile presentation is included in both of the first presentation mode and the second presentation mode, the tactile presentation in the first presentation mode and the tactile presentation in the second presentation mode are different from each other. For example, a tactile presentation element that realizes the tactile presentation in the first presentation mode and a tactile presentation element that realizes the tactile presentation in the second presentation mode may differ in a location or a part, and a method or the type of tactile presentation. In an example where tactile presentation methods are different, tactile presentation in the first presentation mode may be realized by a contact type tactile presentation element, and tactile presentation in the second presentation mode may be realized by a non-contact type tactile presentation element.

The first presentation mode and the second presentation mode may be different presentation modes realized by operating the same presentation element with different operation parameters. For example, the first presentation mode and the second presentation mode that are different from each other may be realized by adjusting the operation parameters such as the strength, the waveform, the length of time, and the interval of vibration described above in the same tactile presentation element.

### - First information and second information

The types of the first information and the second information are not particularly limited. The first information and the second information may be information regarding different types or information regarding the same type.

The type of information may refer to each of driver information, information regarding a surrounding environment, useful information, and information regarding the traveling environment. In this case, a combination of information regarding different types may include an example in which one of the first information and the second information is information regarding the driver state, and the other of the first information or the second information is information regarding traveling environment. A combination of information regarding the same type may include an example in which both the first information and the second information are information regarding the driver state.

The type of information may refer to each piece of information included in the driver information, the information regarding a surrounding environment, the useful information, and the information regarding the traveling environment. In this case, a combination of information regarding different types includes an example in which one of the first information and the second information is information indicating a distance to another vehicle included in the information regarding the traveling environment, and the other of the first information and the second information is information regarding a traveling route included in the information regarding a traveling environment. A combination of information regarding the same type includes an example in which both of the first information and the second information are information indicating a distance to another vehicle included in the information regarding a traveling environment.

Acquisition sources of the first information and the second information may be the same information source, and may be different information sources. For example, information sources of both of the first information and the second information may be the traveling environment sensor 120, an information source of the first information may be the state sensor 110, and an information source of the second information is the car may be a navigation system.

Hereinafter, a description will be mainly made of an example in which the first information is information regarding the driver state, the second information is information regarding the traveling environment, and both of the first presentation mode and the second presentation mode are realized by a tactile presentation element.

If the information regarding the driver state and the information regarding the traveling environment are reported through the same tactile presentation, it is difficult for the driver to recognize which of the information regarding the driver state and the information regarding the traveling environment is reported and the driver may be confused. In contrast, according to the above configuration of the embodiment of the present invention, the driver can accurately and easily recognize which of the information regarding the driver state and the information regarding the traveling environment is reported according to a mode of tactile presentation (information presentation). Hereinafter, a control example by the control unit 150 will be described in more detail.

### - Control example A

As a method of changing a mode of tactile presentation, it is conceivable to change a location of tactile presentation. Locations of a vehicle that the driver comes into contact with are mainly a seat and a steering wheel. Thus, the control unit 150 may cause a vibrator provided in one of the seat and the steering wheel to perform tactile presentation for reporting the information regarding the driver state, and a vibrator provided in the other of the seat and the steering wheel to perform tactile presentation for reporting the information regarding the traveling environment. According to such a configuration, the driver can accurately and easily recognize the reported information according to whether the seat or the steering wheel is vibrated.

### - Control example B

The degree of alertness of a driver tends to decrease during automated driving or in a situation in which automated driving and manual driving are mixed, and in such a situation, the driver often releases his/her hand from a steering wheel. Therefore, it is also useful to change a mode of tactile presentation without using vibration of the steering wheel. From this point of view, the control unit 150 may cause a non-contact tactile presentation element such as the ultrasonic vibration generation device or the air extrusion device described above to perform tactile presentation for reporting information regarding the driver state, and may cause another tactile presentation element such as a vibrator provided in the seat to perform tactile presentation for reporting the information regarding the traveling environment. According to such a configuration, it is possible to reliably notify the driver of the information regarding the driver state even during automated driving or in a situation in which automated driving and manual driving are mixed. It may also be possible to use an airflow generated in a vehicle by opening a window as non-contact type tactile presentation.

### - Control example C

In addition to a seat and a steering wheel, a seat belt is an example of a vehicle location that a driver comes into contact with. Therefore, the control unit 150 may cause a seatbelt winding motor to perform tactile presentation for reporting the information regarding the driver state, and may cause another tactile presentation element such as a vibrator provided in the seat to perform tactile presentation for reporting the information regarding the traveling environment. On the contrary, the control unit 150 may cause the seatbelt winding motor to perform tactile presentation for reporting the information regarding the traveling environment, and may cause another tactile presentation element such as a vibrator provided in the seat to perform tactile presentation for reporting the information regarding the driver state. Since the driver generally wears a seatbelt at all times, even with this configuration, the driver can be reliably notified of the information regarding the driver state during automated driving or in a situation in which automated driving and manual driving are mixed.

### - Control example D

A vibration device may be disposed in a configuration that is not provided in a part of a vehicle. In this case, the control unit 150 may cause the vibration device to perform tactile presentation for reporting the information regarding the driver state, and may cause another tactile presentation element to perform tactile presentation for reporting the information regarding the traveling environment. On the contrary, the control unit 150 may cause the vibrating device to perform tactile presentation for reporting the information regarding the traveling environment, and may cause another tactile presentation element to perform tactile presentation for reporting the information regarding the driver state. Examples of the vibration device disposed in the configuration that is not provided in a part of the vehicle include a vibration device mounted on the driver's hand, and a vibration device disposed to be in close contact with the driver's waist. Such a vibration device may be additionally mounted relatively easily.

### - Control example E

The control unit 150 may use motion acting on the entire vehicle as a tactile presentation mode. For example, the control unit 150 may use a brake system as a tactile presentation element and cause the brake system to intermittently operate a brake such that a driver is presented with a change in acceleration in a front-rear direction of the entire vehicle. The control unit 150 may cause the brake system to perform tactile presentation for reporting the information regarding a driver state, and may cause another tactile presentation element such as a vibrator provided in a seat to perform tactile presentation for reporting the information regarding the traveling environment. On the contrary, the control unit 150 may cause the brake system to perform tactile presentation for reporting the information regarding the traveling environment, and may cause another tactile presentation element such as a vibrator provided in a seat to perform tactile presentation for reporting the information regarding the driver state. The control unit 150 may perform tactile presentation by using a vibrator that is placed on a floor of the vehicle and vibrates the entire vehicle instead of the brake system. As described above, the sense of touch that acts on the entire vehicle and is presented to the driver is clearly different from the sense of touch that acts locally by the vibrator provided in the seat and is presented to the driver. Therefore, the driver can recognize information more accurately and easily.

### <3. Operation>

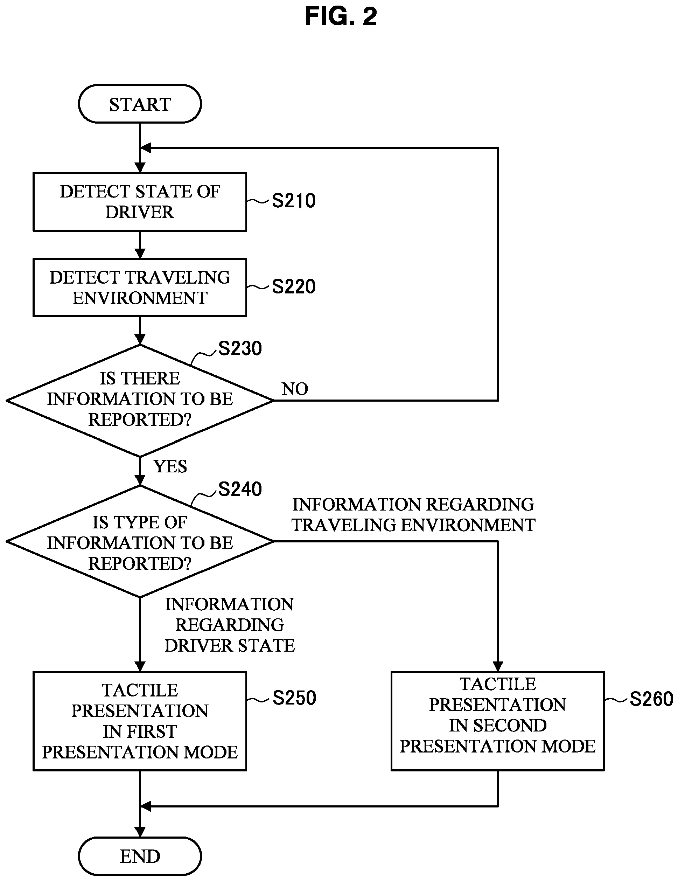

As described above, the configuration of the control system 10 according to the embodiment of the present invention has been described. Next, with reference to FIG. 2, an operation in a tactile presentation control method performed by the control system 10 according to the embodiment of the present invention will be described.

FIG. 2 is a flowchart illustrating an operation of the control system 10 according to the embodiment of the present invention. As illustrated in FIG. 2, the state sensor 110 detects a state of a driver (S210), and the traveling environment sensor 120 detects a traveling environment of a vehicle (S220).

The information acquisition unit 130 attempts to acquire information to be reported to the driver on the basis of a result of the detection by the state sensor 110 and a result of the detection by the traveling environment sensor 120 (S230). Here, the processes from S210 are repeatedly performed while the information to be reported to the driver is not acquired (S230/No). On the other hand, in a case where the information to be reported to the driver is acquired (S230/Yes), the control unit 150 determines the type of information to be reported (S240).

In a case where the type of information to be reported is information regarding the driver state (S240/information regarding driver state), the control unit 150 causes the presentation unit 140 to perform tactile presentation in the first presentation mode (S250). On the other hand, in a case where the type of information to be reported is information regarding the traveling environment

(S240/information regarding traveling environment), the control unit 150 causes the presentation unit 140 to perform tactile presentation in the second presentation mode different from the first presentation mode (S260).

Therefore, the driver can easily recognize whether the reported information is the information regarding the driver state that is an example of the first information or the information regarding the traveling environment that is an example of the second information, according to a mode of tactile presentation.

### <4. Application example>

In the above description, an example in which the control unit 150 causes the presentation unit 140 to perform tactile presentation for the purpose of reporting information has been described, but as an application example, the control unit 150 may further have a function of causing the presentation unit 140 to perform tactile presentation for the purpose of not reporting information. In this case, the control unit 150 causes the presentation unit 140 to perform tactile presentation for reporting the information regarding the driver state and tactile presentation for the purpose of not reporting the information in different modes.

Examples of the tactile presentation for the purpose of not reporting information include tactile presentation with a massage effect (third tactile presentation). In driving for a long time, the blood circulation of the legs and waist that are pressed against the seat deteriorates, which easily leads to fatigue. Therefore, it is effective to perform tactile presentation accompanied by a massage effect during driving from the viewpoint of reducing fatigue.

Specifically, a plurality of vibrators may be provided in a portion of the seat that comes into contact with the driver's feet or waist, and the control unit 150 may repeat control such that the plurality of vibrators are vibrated in order in one direction as tactile presentation with a massage effect. For example, the control unit 150 can promote the blood circulation of the driver while making the driver feel the direction of the vibration by vibrating the plurality of vibrators in order from the back side of the knees to the buttock side.

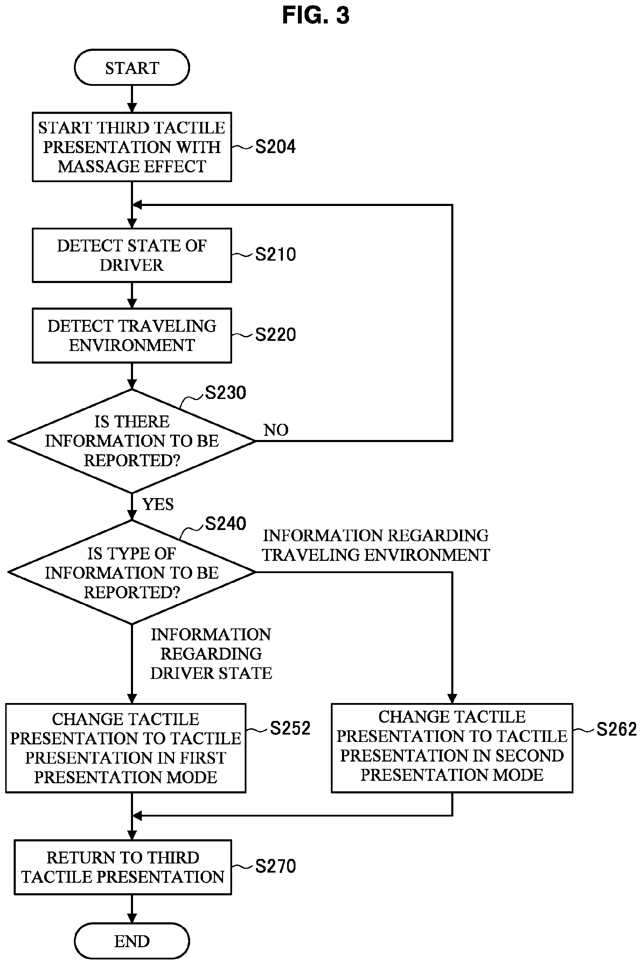

The control unit 150 may change vibration patterns of the plurality of vibrators such that tactile presentation for reporting the information regarding the driver state and tactile presentation for reporting the information regarding the traveling environment can be differentiated from tactile presentation with a massage effect. For example, the control unit 150 may vibrate the plurality of vibrators in an opposite direction in the tactile presentation for reporting the information regarding a traveling environment. The control unit 150 may promote alertness by vibrating the plurality of vibrators in a random order in the tactile presentation for reporting the information regarding the driver state. Hereinafter, an operation according to such an application example will be described with reference to FIG. 3.

FIG. 3 is a flowchart illustrating an operation according to the application example. As illustrated in FIG. 3, the control unit 150 causes the presentation unit 140 to start the third tactile presentation with a massage effect (S204). Thereafter, in a case where the processes in S210 to S240 described with reference to FIG. 2 proceeds and the type of information to be reported is the information regarding the driver state (S240/information regarding driver state), the control unit 150 causes the presentation unit 140 to change the mode of tactile presentation from the third tactile presentation to tactile presentation in the first presentation mode (S252). The driver recognizes that the information regarding the driver state has been reported through the tactile presentation in the first presentation mode.

On the other hand, in a case where the type of information to be reported is the information regarding the traveling environment (S240/information regarding traveling environment), the control unit 150 causes the presentation unit 140 to change the mode of tactile presentation from the third tactile presentation to tactile presentation in the second presentation mode (S262). The driver recognizes that the information regarding the traveling environment has been reported through the tactile presentation in the second presentation mode.

After the process in S252 or the process in S262, the control unit 150 causes the presentation unit 140 to return the tactile presentation to the third tactile presentation (S270).

According to such an application example, the driver can recognize the information regarding the driver state and the information regarding the traveling environment without any trouble while obtaining the blood circulation promoting effect due to a massage.

### <5. Conclusion>

As described above, according to the embodiment of the present invention, it is possible to accurately and easily recognize reported information according to a mode of tactile presentation.

Although the preferred embodiment of the present invention has been described in detail with reference to the accompanying drawings, the present invention is not limited to such examples. It is clear that a person skilled in the art can conceive of various changes or modifications within the scope of the technical ideas described in the claims, and these are also naturally understood to belong to the technical scope of the present invention.

For example, the control unit 150 may make the driver easily recognize information by using other modals such as display or sound in addition to differentiation of a mode of tactile presentation or instead of differentiation of a mode of tactile presentation. For example, the control unit 150 may control display and output of sound in addition to tactile presentation when reporting the information regarding the driver state, and may control tactile presentation and does not have to control display and output of sound when reporting the information regarding the traveling environment. With such a configuration, it is possible to reduce a driver's confusion at the time of information notification. Tactile presentation need not be used in the presentation of any information.

In the above description, the driver state includes a driver's inattentiveness, a driver's distractedness, a driver's unconsciousness, or a driver's drinking condition, but the driver state may include whether or not the driver is a thief of a vehicle. Whether or not the driver is a thief of the vehicle can be determined from, for example, an image or biological information of the driver. In a case where the driver is a thief of the vehicle, the control unit 150 may cause the presentation unit 140 to perform tactile presentation that causes discomfort. According to such a configuration, it is possible to improve a possibility that the thief cannot comfortably use the vehicle, and thus the vehicle can be abandoned early to be returned to an owner. If it is widely recognized that a vehicle cannot be used comfortably even when the vehicle is stolen, an effect of suppressing theft of the vehicle is expected.

In the above description, a distance to another vehicle has been described as the information regarding a traveling environment, but the information regarding a traveling environment may be other information. For example, the information acquisition unit 130 may acquire traveling guide information as the information regarding a traveling environment on the basis of the current position of the vehicle and a preset traveling route. Examples of the traveling guide information include information indicating that a traveling lane needs to be changed for turning left, right, or going straight, and information indicating that a timing of turning left or right is approaching.

In a case where traveling guide information is acquired, the control unit 150 causes the presentation unit 140 to perform tactile presentation for reporting the traveling guide information. For example, the control unit 150 may vibrate the vibrator provided on the steering wheel such that changing of the traveling lane is guided. In addition to the tactile presentation, the control unit 150 may control display of an arrow on a head-up display, blinking of a light emitting portion (for example, an LED) provided in the vehicle, and the like such that the driver is notified that a timing of turning left or turning right approaches. With such a configuration, a time for the driver to look at a navigation screen, that is, a time for the driver to look away from the front is shortened, and thus it is possible to improve the safety of driving.

The information regarding a traveling environment may be information such as a traffic accident rate and a traffic volume at a place near the current position. In a case where information indicating a place where a traffic accident rate or a traffic volume exceeding a threshold value is occurring is acquired, the control unit 150 causes the presentation unit 140 to perform tactile presentation for reporting the sense of direction and the sense of distance of the place. The control unit 150 may more accurately report the sense of direction and the sense of distance of the place by combining display and sound of a center display, a head-up display, or the like. With such a configuration, the driver can know the information related to the place near the current position, and thus the driver can easily take measures for avoiding an accident or a traffic jam.

The information regarding a traveling environment may be information indicating a natural environment near the current position. Examples of the natural environment include the sea, rivers, and mountains. In this case, the control unit 150 controls a mode of presentation performed by the presentation unit 140 according to the information regarding a natural environment near the current position acquired by the information acquisition unit 130. For example, the control unit 150 may control the presentation unit 140 such that tactile presentation and auditory presentation are performed according to a natural environment near the current position. According to such a configuration, the driver can feel a surrounding natural environment more strongly through sensations other than the sense of sight, such as the sense of touch or the sense of hearing, so that the enjoyment of driving is improved.

In the above description, the example in which the first information is the information regarding the driver state and the second information is the information regarding the traveling environment has been mainly described, but other combinations of the first information and the second information may be used. For example, the information acquisition unit 130 may have an operation device that detects an operation, and at least one of the first information and the second information may be information regarding any of the following operations. Alternatively, the first information and the second information may be information regarding an operation corresponding to different items among the following items. The first information and the second information may be different types of information regarding operations corresponding to the same item among the following items. In the following description, an example of information presentation according to an operation corresponding to each item will be described.

### • Pinch-in/pinch-out

In a case where pinch-in or pinch-out is performed on an operation device to enlarge or reduce map display, the control unit 150 may control the presentation unit 140 such that tactile presentation and auditory presentation indicating the pinch-in or the pinch-out are performed. The pinch-in and the pinch-out are operations that change a distance between contact positions of the operation device and two fingers, and, more specifically, the pinch-in is an operation that shortens the distance, and the pinch-out is an operation that lengthens the distance.

### • Swipe

When swipe is performed on the operation device, the control unit 150 may control the presentation unit 140 such that tactile presentation and the auditory presentation indicating the swipe are performed. The swipe is an operation of changing a contact position between the operating device and one finger in a state in which the finger is in contact with the operation device.

### • Operation of list scroll bar

When a scroll operation or a catch operation of a list scroll bar is performed on the operation device, the control unit 150 may control the presentation unit 140 such that tactile presentation and auditory presentation indicating the scroll operation or the catch operation are performed. When the finger is released from the list scroll bar, the control unit 150 may control the presentation unit 140 such that auditory presentation is performed according to the release of the finger. The list scroll bar is an operation region for moving a range to be displayed in a list of a plurality of display items, and includes a linearly disposed main body and a knob located in a part of the main body. The scroll operation is an operation of moving a position of the knob on the main body. Due to the scroll operation, a range to be displayed in the list is moved, that is, scrolling is performed. The catch operation is an operation of stopping movement of a position of the knob on the main body.

### • Character input

When a character input operation is performed on the operation device, the control unit 150 may control the presentation unit 140 such that sound and vibration generated when writing characters with a pen are presented.

### • Movement of display icon

When an operation of moving a display icon is performed on the operation device, the control unit 150 may control the presentation unit 140 such that tactile presentation and auditory presentation indicating the operation are performed. Some data corresponds to the display icon, and the display icon represents the purpose, a function, or the like of the corresponding data with a figure or a pattern.

### • Display icon folder operation

When an operation of putting the display icon in a folder such as a data folder or a trash can folder is performed on the operation device, the control unit 150 may control the presentation unit 140 such that popping sound and vibration generated when an object is dropped into a box are presented. The operation of putting the display icon in the folder is, for example, drag-and-drop. The drag-and-drop is an operation of performing an operation of selecting the display icon and then moving an operation position to a target position while keeping the display icon selected, and thus the display icon is moved to the target position.

### • Operation outside operation valid range

For example, in a dial type operation device, a rotatable range that is an angle range in which a dial is rotatable and an operation valid range in which an operation that is input by rotating the dial is valid may differ. When the rotatable range is wider than the operation valid range and a rotation position of the dial is out of the operation valid range, the control unit 150 may control the presentation unit 140 such that tactile presentation and auditory presentation indicating that the rotation position of the dial is out of the operation valid range are performed.

### • Starting/finishing of application

When an operation of starting or finishing an application is performed on the operation device, the control unit 150 may control the presentation unit 140 such that tactile presentation and auditory presentation indicating starting or finishing of the application are performed.

### • Starting/finishing of voice recognition mode

When an operation of starting or finishing a voice recognition mode is performed on the operation device, the control unit 150 may control the presentation unit 140 such that tactile presentation and auditory presentation indicating starting or finishing of the voice recognition mode are performed. The voice recognition mode is a mode in which a sound collection function that converts a voice that is air vibration into an electrical voice signal and an analysis function that recognizes the content of the voice by analyzing the voice signal are enabled.

### • Pin stabbing on map

When a pin stabbing operation for a map is performed on the operation device, the control unit 150 may control the presentation unit 140 such that tactile presentation and auditory presentation indicating the pin stabbing operation are performed. The pin stabbing operation is an operation of setting a specific point such as a favorite point on the map.

### • Pin selection

When an operation of selecting a pin set on the map is performed on the operation device, the control unit 150 may control the presentation unit 140 such that tactile presentation and auditory presentation indicating the pin selection are performed.

### • Drawing of picture

When an operation of drawing a picture is performed on the operation device, the control unit 150 may control the presentation unit 140 such that sound and vibration generated when drawing a picture with a pen or a brush are presented.

### • Mouse operation

The operation device may be a mouse. When any one of a plurality of buttons provided on the mouse is clicked, the control unit 150 may control the presentation unit 140 such that tactile presentation and auditory presentation corresponding to the clicked button are performed.

In the above description, examples of operations on the operation device that can correspond to the first information or the second information have been described. The control unit 150 may control the presentation unit 140 such that information regarding content such as movies and music is differentiated from other information. When a vehicle door is locked or unlocked with a key, the control unit 150 may control the presentation unit 140 such that tactile presentation and auditory presentation indicating that the door is locked or unlocked with the key are performed. The door may be locked or unlocked through, for example, an operation from a smartphone or a touch operation on a door handle.

The respective steps in the process in the control system 10 of the present specification do not necessarily have to be processed in chronological order in the order described as the flowchart. For example, the respective steps in the process in the control system 10 may be processed in an order different from the order described in the flowchart, or may be processed in parallel.

The respective constituents of the control system 10 may be integrally disposed in one device, or may be separately disposed in two or more devices. For example, a control device having the function of the control unit 150 and a presentation device (presentation system) having the function of the presentation unit 140 may be separately configured, and a control signal may be output from the control device to the presentation device in a wired or wirelessly manner such that the functions and the operations of the control system 10 are realized.

A computer program for causing the hardware such as the CPU, the ROM, and the RAM built in the control system 10 to realize the same function as that of each constituent of the control system 10 described above may be created. A storage medium storing the computer program is also provided.

### Reference Signs List

- 10: control system
- 110: state sensor
- 120: traveling environment sensor
- 130: information acquisition unit
- 140: presentation unit
- 150: control unit

## Claims

1. A control system comprising:
a control unit that controls presentation of information in a presentation unit that presents the information,
wherein the control unit controls a mode of the presentation in the presentation unit according to the information to be reported to a subject.

2. The control system according to claim 1,
wherein the information includes first information and second information, and
wherein the control unit controls presentation of the information in the presentation unit such that the first information and the second information are respectively presented in a first presentation mode and a second presentation mode different from the first presentation mode.

3. The control system according to claim 2,
wherein the presentation unit has a plurality of different types of presentation elements, and
wherein the control unit realizes the first presentation mode and the second presentation mode with different presentation elements.

4. The control system according to claim 2,
wherein the control unit realizes the first presentation mode and the second presentation mode with presentation elements of the same type.

5. The control system according to any one of claims 2 to 4,
wherein the first information and the second information are respectively acquired from different information sources.

6. The control system according to any one of claims 2 to 4,
wherein the first information and the second information are acquired from the same information source.

7. The control system according to any one of claims 2 to 6,
wherein the first information and the second information are the same type of information.

8. The control system according to any one of claims 2 to 6,
wherein the first information and the second information are different types of information.

9. A presentation system comprising:
a presentation unit that presents information,
wherein the presentation unit performs presentation of the information in different modes according to the information to be reported to a subject on the basis of an input control signal.
